(19) Europäisches Patentamt
European Patent Office
Office européen des brevets

(11) **EP 4 617 665 A1**

(12) **EUROPEAN PATENT APPLICATION**

(43) Date of publication:
**17.09.2025 Bulletin 2025/38**

(21) Application number: **24184216.0**

(22) Date of filing: **25.06.2024**

(51) International Patent Classification (IPC):
**G01N 33/574** (2006.01)   **G01N 33/92** (2006.01)

(52) Cooperative Patent Classification (CPC):
**G01N 33/57407; G01N 33/92;** G01N 2800/52

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **14.03.2024 EP 24305387**

(71) Applicants:
• **Centre National de la Recherche Scientifique**
**75016 Paris (FR)**
• **Université Paris Cité**
**75006 Paris (FR)**

• **Institut National de la Santé et de la Recherche Médicale**
**75013 Paris (FR)**

(72) Inventors:
• **BAUD, Véronique**
**75013 PARIS (FR)**
• **BERTHO, Gildas**
**75016 PARIS (FR)**
• **GIRAUD, Nicolas**
**75006 PARIS (FR)**
• **MONTAGNE, Aurélie**
**75006 PARIS (FR)**

(74) Representative: **A.P.I. Conseil**
**Technopôle Hélioparc**
**4, rue Jules Ferry**
**64000 Pau (FR)**

(54) **AN IN VITRO METHOD FOR ESTABLISHING THE PROGNOSIS OF A SUBJECT DIAGNOSED AS SUFFERING OR HAVING SUFFERED FROM A DIFFUSE LARGE B-CELL LYMPHOMA**

(57)     The invention relates to an *in vitro* method for establishing the prognosis of a subject diagnosed as suffering or having suffered from a diffuse large B-cell lymphoma and including:
- an identification step of at least two markers selected from : 2-aminobutyrate or its acid derivative thereof, 2-hydroxybutyrate or its acid derivative thereof, 3-hydroxybutyrate or its acid derivative thereof, LDL-1 lipoprotein, LDL-2 lipoprotein, LDL-1-CH lipoprotein, LDL-1 lipoprotein phospholipids, the Apo-B subfraction of LDL-1 lipoprotein, LDL-1 lipoprotein triglycerides, LDL-2 lipoprotein triglycerides, LDL-3 lipoprotein triglycerides, formic acid and acetyl acetic acid, identifying said at least two markers being the prognosis of, or a risk of, a bad clinical course of the diffuse large B-cell lymphoma in the subject.

Figure 3

**Description**

**FIELD OF THE INVENTION**

The present invention relates to the field of medicine. More particularly the invention relates to a method for establishing the prognosis of a subject of, or a risk of, a bad clinical course of the diffuse large B-cell lymphoma (DLBCL) based on the analysis of a sample of a biological fluid from said subject. The inventors have indeed identified a combination of markers, which when identified in a sample of a biofluid from a subject in a subject suffering or having suffered from a DLBCL is related to a bad evolution of the DLBCL in said patient over time. Said combination comprises at least two markers of a list of thirteen relevant markers. Such information provides valuable information for disease management, since it allows to predict a risk for a relapse particularly in said subject.

**BACKGROUND OF THE INVENTION**

Metabolic dysregulation is now recognized as one of the major markers of cancer in humans. However, they remain very poorly characterized in B lymphomas.

Diffuse large B-cell lymphoma (DLBCL) is the most common lymphoma, representing approximately a third of all lymphoma cases worldwide (18,000 new cases/year in France). Diffuse large B-cell lymphoma (DLBCL) accounts for approximately one-third of non-Hodgkin's lymphomas. While some DLBCL patients are cured with traditional chemotherapy, the remainder die from the disease.

Despite the introduction of immunotherapy (anti-CD20, Rituximab) together with conventional chemotherapies, 40% of patients with DLBCL will relapse, be refractory and die. Even though cutting-edge immunotherapies (CAR-T cells, bispecific antibodies) have revolutionized the management of refractory or relapsed DLBCL patients, 60% of them will relapse or will not respond to treatments.

Patent document WO2017053555 address a solution of treating, preventing and managing cancer including DLBCL, particularly cancers that are refractory to standard treatments, such as surgery, radiation therapy, chemotherapy and hormonal therapy, while reducing or avoiding the toxicities and/or side effects associated with the conventional therapies.

To that end, it has been proposed a method for predicting the responsiveness of a diffuse large B-cell lymphoma (DLBCL) patient to treatment with a drug which includes determining the level of expression of a plurality of genes in a biological sample and comparing them to the level of expression of the same genes in biological sample(s) from DLBCL patient(s) known to be responsive or not responsive to the drug. The similarity of expression of the genes relative to the level of expression of the genes biological sample from subject known to be responsive or not indicates that the DLBCL in the first patient will be responsive or not to treatment with the drug. However, this method is not adapted to establish a prognosis on whether the patient, in remission from a DLBCL, is at risk of relapsing which is a major issue to improve the long-term survival chances of the patient by implementing appropriate treatments before the DLBCL reappears and spreads. The current International Prognostic Index (IPI) used in predicting the prognosis of patients with aggressive non-Hodgkin's lymphoma does not allow this prognosis either.

**SUMMARY OF THE INVENTION**

The inventors surprisingly found that a set of markers which when a combination of them is identified in a biological sample from a patient having been affected by or suffering from a DLBCL allows to establish a risk of relapse and a prognosis of survival. More particularly, inventors identified specific markers which allow to discriminate group of patients with a high risk of relapse and a low probability of survival whereas it was impossible to discriminate said groups using the International Prognostic Index for Diffuse Large B-cell currently used today.

Further, levels of said markers are easily measurable in easy to collect biological sample allowing thus to conduct more frequent routine analyses and to implement appropriate treatment more adaptively before the reappearance of the DLBCL. Combinations of markers of the invention are of a great interest as they unlock new possibilities of prognosis of DLBCL relapse in high-risk patient for which diagnosis meets a very strong medical need.

Accordingly, a first object of the invention relates to an *in vitro* method for establishing the prognosis of a subject diagnosed as suffering or having suffered from a diffuse large B-cell lymphoma from at least one sample of biological fluid, the prognosis method including:

- an identification step, in the at least one sample of biological fluid, of at least two markers selected from : 2-aminobutyrate or its acid derivative thereof, 2-hydroxybutyrate or its acid derivative thereof, 3-hydroxybutyrate or its acid derivative thereof, LDL-1 lipoprotein, LDL-2 lipoprotein, LDL-1-CH lipoprotein, LDL-1 lipoprotein phospholipids, the Apo-B subfraction of LDL-1 lipoprotein, LDL-1 lipoprotein triglycerides, LDL-2 lipoprotein triglycerides, LDL-3 lipoprotein triglycerides, formic acid and acetyl acetic acid,

identifying said at least two markers being the prognosis of, or a risk of, a bad clinical course of the diffuse large B-cell lymphoma in the subject.

The method for establishing the prognosis of a subject diagnosed as suffering or having suffered from a diffuse large B-cell lymphoma according to the invention may comprise the following optional features :

- The at least two markers comprise 2-aminobutyrate or its acid derivative thereof in combination with at least a marker selected from : 2-hydroxybutyrate or its acid derivative thereof, 3-hydroxybutyrate or its acid derivative thereof, LDL-1 lipoprotein, LDL-2 lipoprotein, LDL-1-CH lipoprotein, LDL-1 lipoprotein phospholipids, the Apo-B subfraction of LDL-1 lipoprotein, LDL-1 lipoprotein triglycerides, LDL-2 lipoprotein triglycerides, LDL-3 lipoprotein triglycerides, formic acid and acetyl acetic acid.
- The at least two markers comprise LDL-1 lipoprotein in combination with at least a marker selected from : 2-aminobutyrate or its acid derivative thereof, 2-hydroxybutyrate or its acid derivative thereof, 3-hydroxybutyrate or its acid derivative thereof, LDL-2 lipoprotein, LDL-1-CH lipoprotein, LDL-1 lipoprotein phospholipids, the Apo-B subfraction of LDL-1 lipoprotein, LDL-1 lipoprotein triglycerides, LDL-2 lipoprotein triglycerides, LDL-3 lipoprotein triglycerides, formic acid and acetyl acetic acid.
- The at least two markers comprise 3-hydroxybutyrate or its acid derivative thereof in combination with at least a marker selected from : 2-aminobutyrate or its acid derivative thereof, 2-hydroxybutyrate or its acid derivative thereof, LDL-1 lipoprotein, LDL-2 lipoprotein, LDL-1-CH lipoprotein, LDL-1 lipoprotein phospholipids, the Apo-B subfraction of LDL-1 lipoprotein, LDL-1 lipoprotein triglycerides, LDL-2 lipoprotein triglycerides, LDL-3 lipoprotein triglycerides, formic acid and acetyl acetic acid.
- The at least two markers comprise 2-aminobutyrate or its acid derivative thereof and LDL-1 lipoprotein optionally in combination with at least a marker selected from : 2-hydroxybutyrate or its acid derivative thereof, 3-hydroxybutyrate or its acid derivative thereof, LDL-2 lipoprotein, LDL-1-CH lipoprotein, LDL-1 lipoprotein phospholipids, the Apo-B subfraction of LDL-1 lipoprotein, LDL-1 lipoprotein triglycerides, LDL-2 lipoprotein triglycerides, LDL-3 lipoprotein triglycerides, formic acid and acetyl acetic acid.
- The at least two markers comprise 2-aminobutyrate or its acid derivative thereof and 3-hydroxybutyrate or its acid derivative thereof optionally in combination with at least a marker selected from : 2-hydroxybutyrate or its acid derivative thereof, LDL-1 lipoprotein, LDL-2 lipoprotein, LDL-1-CH lipoprotein, LDL-1 lipoprotein phospholipids, the Apo-B subfraction of LDL-1 lipoprotein, LDL-1 lipoprotein triglycerides, LDL-2 lipoprotein triglycerides, LDL-3 lipoprotein triglycerides, formic acid and acetyl acetic acid.
- The at least two markers comprise LDL-1 lipoprotein and 3-hydroxybutyrate or its acid derivative thereof optionally in combination with at least a marker selected from : 2-aminobutyrate or its acid derivative thereof, 2-hydroxybutyrate or its acid derivative thereof, LDL-2 lipoprotein, LDL-1-CH lipoprotein, LDL-1 lipoprotein phospholipids, the Apo-B subfraction of LDL-1 lipoprotein, LDL-1 lipoprotein triglycerides, LDL-2 lipoprotein triglycerides, LDL-3 lipoprotein triglycerides, formic acid and acetyl acetic acid.
- The at least two markers comprise 2-aminobutyrate or its acid derivative thereof, LDL-1 lipoprotein and 3-hydroxybutyrate or its acid derivative thereof optionally in combination with at least a marker selected from : 2-hydroxybutyrate or its acid derivative thereof, LDL-2 lipoprotein, LDL-1-CH lipoprotein, LDL-1 lipoprotein phospholipids, the Apo-B subfraction of LDL-1 lipoprotein, LDL-1 lipoprotein triglycerides, LDL-2 lipoprotein triglycerides, LDL-3 lipoprotein triglycerides, formic acid and acetyl acetic acid.
- a step of dosing the at least two identified markers and comparing to a predetermined level threshold so as to determine a difference for each of the identified markers, said determination being the prognosis of, or a risk of, a bad clinical course of the diffuse large B-cell lymphoma in the subject.
- The 2-aminobutyrate or its acid derivative thereof is at a concentration superior or equal to 14 $\mu$mol/L.
- The LDL-1 lipoprotein is at a concentration superior or equal to 227 nmol/L.
- The bad clinical course is selected from : an impaired 5-year survival, an impaired progression-free survival and/or an increased risk of death.
- The at least one sample of biological fluid is selected from : blood, plasma, serum, urine, sweat and/or saliva, more preferably from plasma or urine.
- The subject is a mammal, more preferably a human subject.

According to a second object, the invention relates to a method of treating a diffuse large B-cell lymphoma in a subject diagnosed as suffering or having a risk of relapse comprising :

- from at least one sample of biological fluid :

◦ an identification step, in the at least one sample of biological fluid, of at least two markers selected from : 2-aminobutyrate or its acid derivative thereof, 2-hydroxybutyrate or its acid derivative thereof, 3-hydroxybutyrate or its acid derivative thereof, LDL-1 lipoprotein, LDL-2 lipoprotein, LDL-1-CH lipoprotein, LDL-1 lipoprotein phospholipids, the Apo-B subfraction of LDL-1 lipoprotein, LDL-1 lipoprotein triglycerides, LDL-2 lipoprotein triglycerides, LDL-3 lipoprotein triglycerides, formic acid and acetyl acetic acid,

identifying said at least two markers being the prognosis of, or a risk of, a bad clinical course of the diffuse large B-cell lymphoma in the subject, and
a step of adapting the treatment of said subject, including in particular an adaptation of the monitoring rhythm, the administration of an autologous stem cell transplant, the administration of immunomodulator agents (e.g. Golcadomide or Lenaledomide) in combination or not with R-CHOP treatment, administration of an immunotherapy, for example a CAR-T cell therapy (e.g. anti-CD19 or Allo-CART), administration of antibodies (e.g. bispecific anti-CD3 and anti-CD19 antibodies such as blinatumomab), and/or the administration of palliative chemotherapy.
According to a third object, the invention relates to a system for establishing the prognosis of a subject diagnosed as suffering or having suffered from a diffuse large B-cell lymphoma from at least one sample of biological fluid, the system comprising an identification device, at least one processor, a referenced DLBCL sample database comprising:

◦ plasma levels, of each sample, of markers : 2-aminobutyrate or its acid derivative thereof, 2-hydroxybutyrate or its acid derivative thereof, 3-hydroxybutyrate or its acid derivative thereof, LDL-1 lipoprotein, LDL-2 lipoprotein, LDL-1-CH lipoprotein, LDL-1 lipoprotein phospholipids, the Apo-B subfraction of LDL-1 lipoprotein, LDL-1 lipoprotein triglycerides, LDL-2 lipoprotein triglycerides, LDL-3 lipoprotein triglycerides, formic acid and acetyl acetic acid,
◦ group of referenced DLBCL samples, each group comprising all referenced DLBCL samples for which plasma levels of at least two corresponding markers are associated to a same survival indicator value over time,

in which:

- the identification device is configured to determine the composition of the biological fluid sample, said composition comprising at least two markers selected from : 2-aminobutyrate or its acid derivative thereof, 2-hydroxybutyrate or its acid derivative thereof, 3-hydroxybutyrate or its acid derivative thereof, LDL-1 lipoprotein, LDL-2 lipoprotein, LDL-1-CH lipoprotein, LDL-1 lipoprotein phospholipids, the Apo-B subfraction of LDL-1 lipoprotein, LDL-1 lipoprotein triglycerides, LDL-2 lipoprotein triglycerides, LDL-3 lipoprotein triglycerides, formic acid and acetyl acetic acid,
- the processor is configured to:

◦ calculate a concentration for each marker of the at least two markers identified in the composition,
◦ compare the calculated concentration of each identified marker to respective predetermined level thresholds to determine an upward increase of each identified marker,
◦ determine, from the referenced DLBCL sample database, at least one sample comprising the same markers, of the at least two markers identified in the composition, for which the upward increase relative to the respective predetermined level thresholds is the closest,
◦ generate a risk indicator based on the survival indicator associated to the group from which the at least one sample comprising the same markers is determined.

According to a fourth object, the invention relates to an *in vitro* method for establishing the prognosis of a subject diagnosed as suffering or having suffered from a diffuse large B-cell lymphoma from at least one sample of biological fluid, the prognosis method including:

- an identification step, in the at least one sample of biological fluid, of at least two markers selected from : 2-aminobutyrate or its acid derivative thereof, 2-hydroxybutyrate or its acid derivative thereof, 3-hydroxybutyrate or its acid derivative thereof, LDL-1 lipoprotein, LDL-2 lipoprotein, LDL-1-CH lipoprotein, LDL-1 lipoprotein phospholipids, the Apo-B subfraction of LDL-1 lipoprotein, LDL-1 lipoprotein triglycerides, LDL-2 lipoprotein triglycerides, LDL-3 lipoprotein triglycerides, formic acid and acetyl acetic acid,

identifying said at least two markers being the prognosis of, or a risk of, a bad clinical course of the diffuse large B-cell lymphoma in the subject.

**LEGEND OF DRAWINGS**

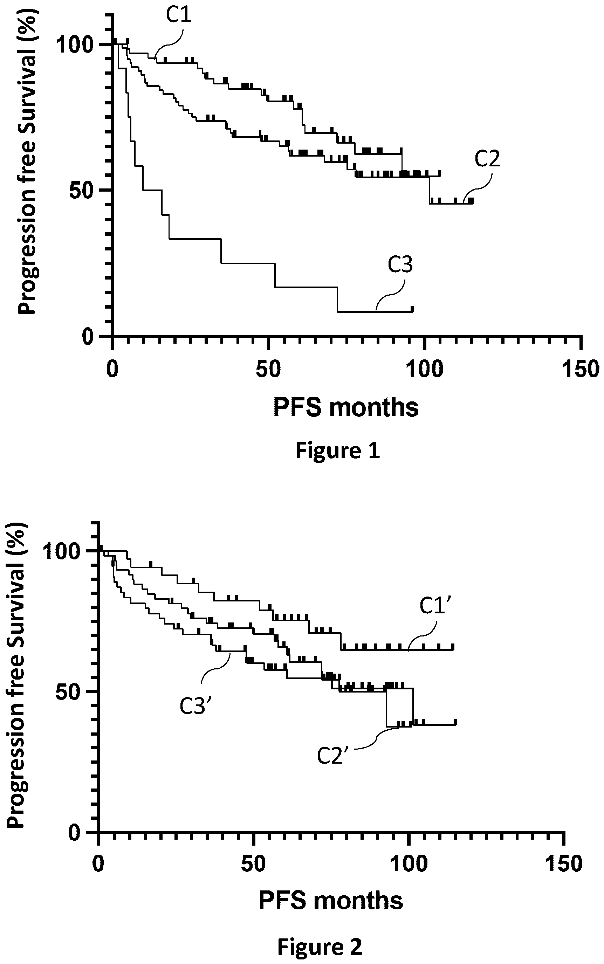

**[Fig. 1] Figure 1** represents a Kaplan-Meier plot of Progression Free Survival (PFS) for DLBCL patients from the REMARC cohort, n=154. Risk categories according to plasma levels of 2-aminobutyrate and LDL-1 lipoprotein as evaluated by NMR prior to any treatment. C1, C2 and C3 curves represent low risk (n=62), intermediate risk (n=79) and high risk (n=13) patients, respectively. P value < 0.0001 calculated by Log-rank (Mantel-Cox, <https://kmplot. com>).

**[Fig. 2] Figure 2** represents a Kaplan-Meier plot of Progression Free Survival (PFS) for DLBCL patients from the REMARC cohort, n=154 according to the International Prognosis Index (IPI). C1', C2' and C3' curves represent low risk (n=35), intermediate risk (n=61) and high risk (n=57) patients, respectively. P value = 0.1705 calculated by Log-rank (Mantel-Cox, <https://kmplot.com>).

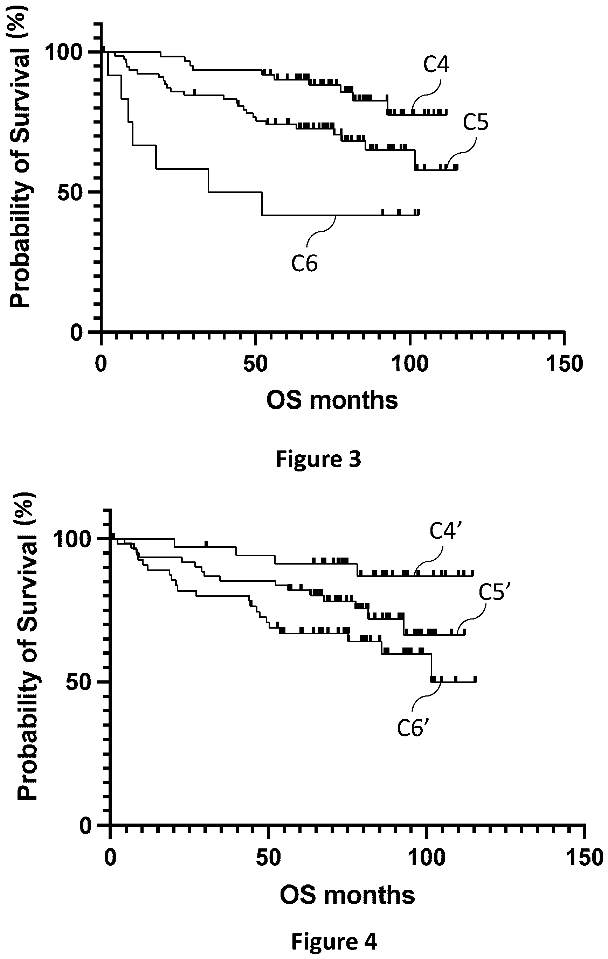

**[Fig. 3] Figure 3** represents a Kaplan-Meier plot of Overall Survival (OS) for DLBCL patients from the REMARC cohort, n=154. Risk categories according to plasma levels of 2-aminobutyrate and LDL-1 lipoprotein as evaluated by NMR prior to any treatment. C4, C5 and C6 curves represent low risk (n=62), intermediate risk (n=79) and high risk (n=13) patients, respectively. P value = 0.0015 calculated by Log-rank (Mantel-Cox, <https://kmplot.com>).

**[Fig. 4] Figure 4** represents a Kaplan-Meier plot of Overall Survival (OS) for DLBCL patients from the REMARC cohort, n=154 according to the International Prognosis Index (IPI). C4', C5' and C6' curves represent low risk (n=35), intermediate risk (n=61) and high risk (n=57) patients, respectively. P value = 0.0152 calculated by Log-rank (Mantel-Cox, <https://kmplot.com>).

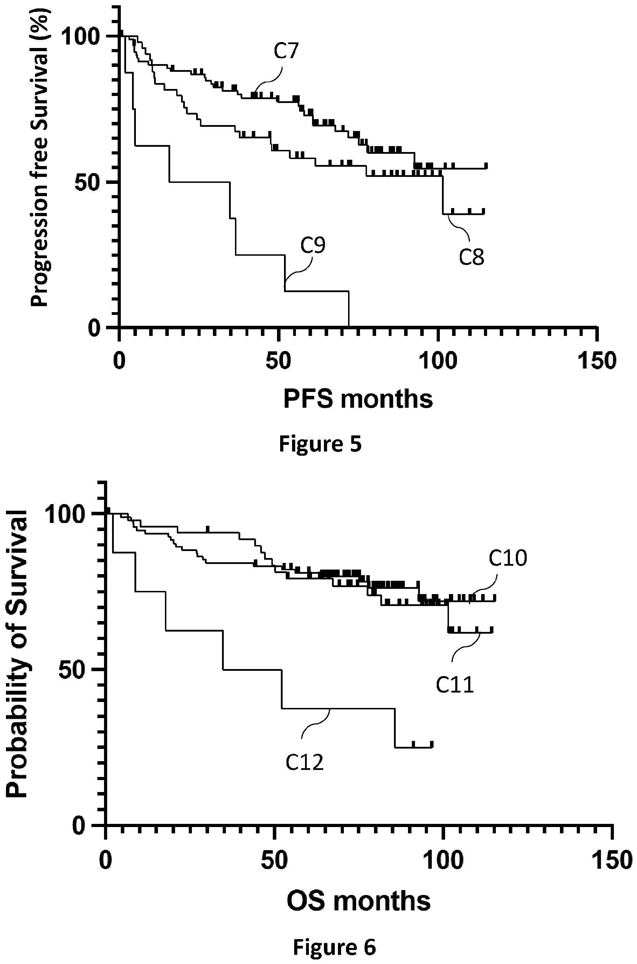

**[Fig. 5] Figure 5** represents a Kaplan-Meier plot of Progression Free Survival (PFS) for DLBCL patients from the REMARC cohort, n=154. Risk categories according to plasma levels of 2-aminobutyrate and 3-hydroxybutyrate as evaluated by NMR prior to any treatment. C7, C8 and C9 curves represent low risk (n=96), intermediate risk (n=50) and high risk (n=8) patients, respectively. P value < 0.0001 calculated by Log-rank (Mantel-Cox, <https://kmplot. com>).

**[Fig. 6] Figure** 6 represents a Kaplan-Meier plot of Overall Survival (OS) for DLBCL patients from the REMARC cohort, n=154. Risk categories according to plasma levels of 2-aminobutyrate and 3-hydroxybutyrate as evaluated by NMR prior to any treatment. C10, C11 and C12 curves represent low risk (n=96), intermediate risk (n=50) and high risk (n=8) patients, respectively. P value = 0.0014 calculated by Log-rank (Mantel-Cox, <https://kmplot.com>).

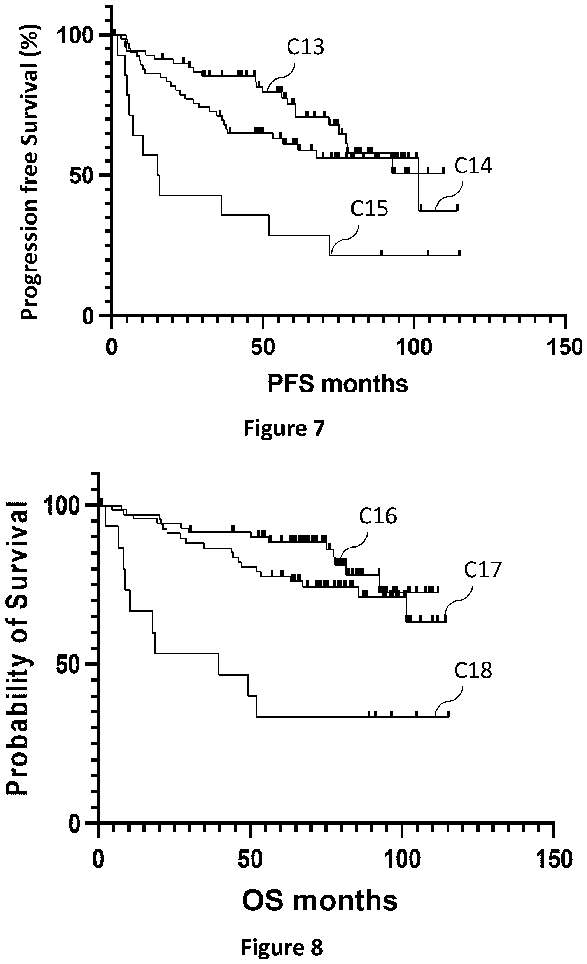

**[Fig. 7] Figure 7** represents a Kaplan-Meier plot of Progression Free Survival (PFS) for DLBCL patients from the REMARC cohort, n=154. Risk categories according to plasma levels of 2-aminobutyrate, LDL-1 lipoprotein and 2 hydroxybutyrate as evaluated by NMR prior to any treatment. C13, C14 and C15 curves represent low risk (n=71), intermediate risk (n=68) and high risk (n=15) patients, respectively. P value = 0.011 calculated by Log-rank (Mantel-Cox, <https://kmplot.com>).

**[Fig. 8] Figure 8** represents a Kaplan-Meier plot of Overall Survival (OS) for DLBCL patients from the REMARC cohort, n=154 according to plasma levels of 2-aminobutyrate, LDL-1 lipoprotein and 2 hydroxybutyrate as evaluated by NMR prior to any treatment. C16, C17 and C18 curves represent low risk (n=71), intermediate risk (n=68) and high risk (n=15) patients, respectively. P value < 0.0001 calculated by Log-rank (Mantel-Cox, <https://kmplot.com>).

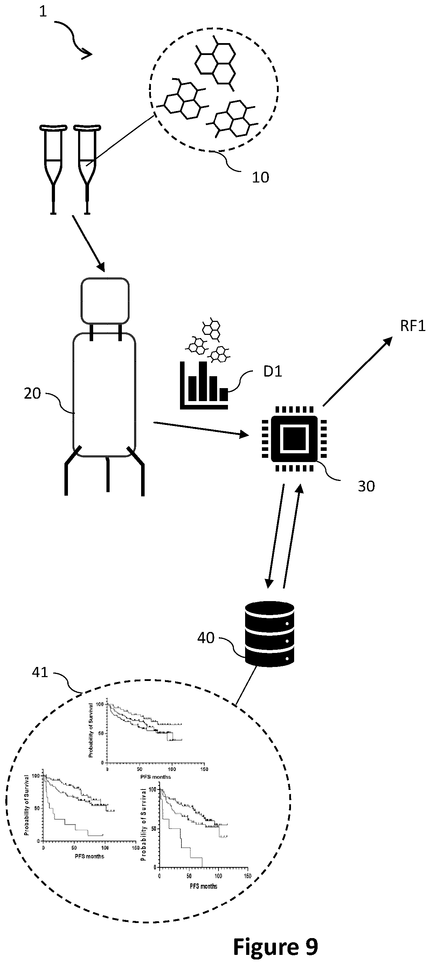

**[Fig. 9] Figure 9** is an illustration of a representation of a system for establishing the prognosis of a subject diagnosed as suffering or having suffered from a diffuse large B-cell lymphoma from at least one sample of biological fluid according to the invention.

## DETAILED DESCRIPTION OF THE INVENTION

### Definitions

As intended herein, the term "comprising" has the meaning of "including" or "containing", which means that when an object "comprises" one or several elements, other elements than those mentioned may also be included in the object. In contrast, when an object is said to "consist of" one or several elements, the object cannot include other elements than those mentioned.

**"DLBCL"** as used herein refers to a diffuse large B-cell lymphoma.

As used herein, the term **"subject"** refers to any mammal, e.g. mouse, rat, monkey, dog, human, preferably a human. Preferably, it refers to any mammal, e.g. mouse, rat, monkey, dog, human, thought to develop or suspected of suffering from DLBCL, or having suffered from DLBCL. More preferably it refers to a human thought to develop or suspected of suffering from DLBCL or having suffered from DLBCL. A subject having suffered form DLBCL is a subject that experienced DLBCL, has been treated for and present no sign of the disease and/or is a in remission phase.

**"Biological sample"** is thought to include any sample from the subject. In a preferred embodiment, a biological sample refers to body fluids as blood, plasma, serum, urine, sweat and/or saliva, more preferably from plasma or urine. Biological sample is thought to designate a raw sample from the subject or a sample resulting from any pre-treatment step of the said sample, such as, for example, a filtration, a centrifugation, an addition of a reagent, a fractionation or any other treatment aiming at facilitating the detection and or identification of the markers of the invention.

As used herein, **"treatment"** or **"to treat"** includes the therapy, the prevention, the prophylaxis, the retardation or the reduction of symptoms provoked by or of the causes of a disease. When related to DLBCL **"treatment"** or **"to treat"** more particularly includes stopping, stabilizing or slowing down the progression of a disease, or lessening the severity of its symptoms. In other words, it encompasses the eradication of a tumour, decreasing the size of a tumour, stopping or slowing down the development of tumour. The terms "treatment" or "to treat" also encompasses therapy, prevention, prophylaxis, retardation or reduction of symptoms of the disease in a subject who has been subjected to a treatment for DLBCL and who, after a remission period, is experiencing or suspected to experience relapse. Also, "treatment" or "to treat" encompass therapy, prevention, prophylaxis, retardation or reduction of symptoms of cancer refractory to current treatments as e.g. chemotherapeutic treatment, immunotherapies, anti-metabolic drugs targeting mitochondrial metabolism, anti-glycolytic therapies.

The expression **"identification of a combination of markers"** as used herein can include co-identification of different markers according to the invention in a single biological sample of a subject. The markers can also be identified separately from a same biological sample or they can be identified separately from different biological samples from the subject. For example, one of the markers of the combination of markers can be identified in a biological sample which is a sample of urine and one of the other markers of the combination can be identified in another biological sample which is a sample of blood plasma.

As used herein, a named marker or drug is thought to designate said chemical marker or drug as named in the application, as well as any pharmaceutically acceptable salt, hydrate, stereoisomer, racemate, conjugate, pro-drug thereof, of any purity.

The expression " **Apo-B subfraction of LDL-1 lipoprotein"** corresponds to the Apo-B100 sub fraction of Apo-B. ApoB-100 is a protein that corresponds to the corresponding to complete translation of mRNA of *APOB* gene.

By **"relapse"** it may be understood the appearance of new tumour lesions, for example after the completion of treatment and/or a period of remission.

The expression « **survival indicator** » may be understood as a probability of an occurrence or a non-occurrence of a predetermined event in a patient over a predetermined time. As non-limitative examples, a survival indicator may be a Progression free Survival indicator, an Overall Survival indicator, a Disease Free-Survival indicator or more generally any kind of indicators well-known in evaluating the evolution of a disease, more particularly in oncology.

### Methods of classifying/identifying patient with a risk of relapse from DLBCL

As exemplified in the experimental data, a list of markers has been found to be useful to provide a prognosis of the risk of DLBCL relapse for patient with a low probability of survival. Based on these markers, it is now possible to discriminate a group of patients at high risk of relapsing and dying from patients showing lesser risk of relapse and better chance of survival over time. Accordingly, identifying these markers allow to help making a diagnosis ahead of the DLBCL reappearance which implies that treatments or specific disease management can be implemented in patients at risk in such a way that it improves their chance of survival, for example by adapting the follow up in said subject, and/or administering a treatment even though no clinical sign of relapse is obviously detected.

Accordingly, a first object of the invention is an *in vitro* method for establishing the prognosis of a subject diagnosed as suffering or having suffered from a diffuse large B-cell lymphoma (DLBCL) from at least one sample of biological fluid which includes an identification step of at least two markers in the at least one sample of biological fluid.

In the invention, the at least two markers identified are selected from : 2-aminobutyrate, 2-hydroxybutyrate, 3-hydroxybutyrate, LDL-1 lipoprotein, LDL-2 lipoprotein, LDL-1-CH lipoprotein, LDL-1 lipoprotein phospholipids, the Apo-B subfraction of LDL-1 lipoprotein, LDL-1 lipoprotein triglycerides, LDL-2 lipoprotein triglycerides, LDL-3 lipoprotein triglycerides, formic acid and acetyl acetic acid.

Identifying the at least two markers previously mentioned is associated with the prognosis of, or a risk of, a bad clinical course of the diffuse large B-cell lymphoma (DLBCL) in the subject. Indeed, as it will be shown in the examples, the mere identification of at least two of these markers in at least a sample of a biofluid of the subject is found sufficient to classify the subject at risk of DLBCL relapse or with low chance of survival.

A bad clinical course, according to the invention, may be selected from : an impaired 5-year survival, an impaired progression-free survival and/or an increased risk of death.

In an embodiment, at least 2, 3 ,4, 5, 6 or even more of the above markers are identified in the method of the invention.

According to an embodiment of the invention, the at least two markers of the *in vitro* method according to the invention comprise 2-aminobutyrate in a combination with at least a marker selected from : 2-hydroxybutyrate, 3-hydroxybutyrate, LDL-1 lipoprotein, LDL-2 lipoprotein, LDL-1-CH lipoprotein, LDL-1 lipoprotein phospholipids, the Apo-B subfraction of LDL-1 lipoprotein, LDL-1 lipoprotein triglycerides, LDL-2 lipoprotein triglycerides, LDL-3 lipoprotein triglycerides, formic acid and acetyl acetic acid or a combination thereof.

According to another embodiment of the invention, the at least two markers of the *in vitro* method comprise LDL-1 lipoprotein in a combination with at least a marker selected from : 2-aminobutyrate, 2-hydroxybutyrate, 3-hydroxybutyrate, LDL-2 lipoprotein, LDL-1-CH lipoprotein, LDL-1 lipoprotein phospholipids, the Apo-B subfraction of LDL-1 lipoprotein, LDL-1 lipoprotein triglycerides, LDL-2 lipoprotein triglycerides, LDL-3 lipoprotein triglycerides, formic acid and acetyl acetic acid or a combination thereof.

According to another embodiment of the invention, the at least two markers of the *in vitro* method comprise 3-hydroxybutyrate in a combination with at least a marker selected from : 2-aminobutyrate, 2-hydroxybutyrate, LDL-1 lipoprotein, LDL-2 lipoprotein, LDL-1-CH lipoprotein, LDL-1 lipoprotein phospholipids, the Apo-B subfraction of LDL-1 lipoprotein, LDL-1 lipoprotein triglycerides, LDL-2 lipoprotein triglycerides, LDL-3 lipoprotein triglycerides, formic acid and acetyl acetic acid or a combination thereof.

According to further embodiment of the invention, the at least two markers of the *in vitro* method comprise 2-aminobutyrate and LDL-1 lipoprotein optionally in a combination with at least a marker selected from : 2-hydroxybutyrate, 3-hydroxybutyrate, LDL-2 lipoprotein, LDL-1-CH lipoprotein, LDL-1 lipoprotein phospholipids, the Apo-B subfraction of LDL-1 lipoprotein, LDL-1 lipoprotein triglycerides, LDL-2 lipoprotein triglycerides, LDL-3 lipoprotein triglycerides, formic acid and acetyl acetic acid or a combination thereof.

According to another embodiment of the invention, the at least two markers of the *in vitro* method comprise 2-aminobutyrate and 3-hydroxybutyrate optionally in a combination with at least a marker selected from : 2-hydroxybutyrate, LDL-1 lipoprotein, LDL-2 lipoprotein, LDL-1-CH lipoprotein, LDL-1 lipoprotein phospholipids, the Apo-B subfraction of LDL-1 lipoprotein, LDL-1 lipoprotein triglycerides, LDL-2 lipoprotein triglycerides, LDL-3 lipoprotein triglycerides, formic acid and acetyl acetic acid or a combination thereof.

According to another embodiment of the invention, the at least two markers of the *in vitro* method comprise LDL-1 lipoprotein and 3-hydroxybutyrate optionally in combination with at least a marker selected from : 2-aminobutyrate, 2-hydroxybutyrate, LDL-2 lipoprotein, LDL-1-CH lipoprotein, LDL-1 lipoprotein phospholipids, the Apo-B subfraction of LDL-1 lipoprotein, LDL-1 lipoprotein triglycerides, LDL-2 lipoprotein triglycerides, LDL-3 lipoprotein triglycerides, formic acid and acetyl acetic acid or a combination thereof.

According to another embodiment of the invention, the at least two markers of the *in vitro* method comprise 2-aminobutyrate, LDL-1 lipoprotein and 3-hydroxybutyrate optionally in combination with at least a marker selected from : 2-hydroxybutyrate, LDL-2 lipoprotein, LDL-1-CH lipoprotein, LDL-1 lipoprotein phospholipids, the Apo-B subfraction of LDL-1 lipoprotein, LDL-1 lipoprotein triglycerides, LDL-2 lipoprotein triglycerides, LDL-3 lipoprotein triglycerides, formic acid and acetyl acetic acid or a combination thereof.

In an embodiment of the invention, the at least two markers can be identified respectively in a different biological sample or in a same one. More preferably, the at least two markers are identified in the same biological sample or a fraction of the same biological sample. In the invention, it is expected that more than two markers can be identified, accordingly, one or several markers can be identified in a biological sample while one or more other markers can be identified in one or more other biological samples. More preferably all the markers are identified in a single biological sample or different fractions thereof.

Said biological sample is preferably a biological fluid, most of them being of an easy collection. For example, the biological fluid may be selected from: blood, plasma, serum, urine, sweat and/or saliva, more preferably from plasma or urine.

As referred in the invention, the subject may be a mammal, more preferably the subject is a human.

Accordingly, the method of the invention can further comprise a step of dosing the at least two identified markers, for example measuring the level of each of identified markers, and comparing it to a predetermined a level threshold, so as to determine a the relative level of the identified markers in regard with said threshold (i.e. being above or below said threshold), said determination being the prognosis of, or a risk of, a bad clinical course of the diffuse large B-cell lymphoma in the subject.

Said threshold or level, can be a concentration, a ratio in regard to another species, or an absolute quantity for said marker.

The step of identifying or dosing the at least two markers can be implemented by known techniques such as Liquid Chromatography-Mass Spectrometry, Gas Chromatography-Mass Spectrometry, Nuclear Magnetic Resonance Spectroscopy or Capillary Electrophoresis-Mass Spectrometry which are nowadays more and more currently used in medical analysis laboratory. Each marker can be associated with a predetermined level threshold.

In an embodiment of the invention, the difference can be a positive or a negative variation, more particularly it is a positive variation in regard with the predetermined level threshold. Said negative or positive variation is found associated with the prognosis of, or a risk of, a bad clinical course of the diffuse large B-cell lymphoma in the subject. In other words, the marker can be found at a level lower than said threshold or at a level higher than said threshold.

Said threshold can correspond to the upper or the lower value found in a group of subjects with a bad clinical course, or conversely with a good clinical course. Said threshold can also correspond to the mean level found in a non-diseased population.

Also, said threshold can correspond to a mean level found in the same subject during previous analyses, an increase of the level in regard with said threshold being associated with the prognosis of, or a risk of, a bad clinical course of the diffuse large B-cell lymphoma.

As an illustrative example, when the at least two markers comprise 2-aminobutyrate, the prognosis of, or a risk of, a bad clinical course of the diffuse large B-cell lymphoma in the subject is characterized by a 2-aminobutyrate concentration superior or equal to 14 μmol/L, preferably superior to 14 μmol/L and inferior to 52 μmol/L, and more preferably superior or equal to 52 μmol/L in a plasma sample of the subject.

As an alternative or complement, when the at least two markers comprise LDL-1 lipoprotein, the prognosis of, or a risk of, a bad clinical course of the diffuse large B-cell lymphoma in the subject is characterized by a LDL-1 lipoprotein concentration superior or equal to 227 nmol/L in a plasma sample.

According to one aspect of the invention, it is provided a method for generating a risk indicator. The inventors developed a method which calculates a risk indicator through an integration of multiple inputs (including at least 2 markers from the 13 markers of the invention). Each input represents a quantified marker and/or a survival indicator and is preferably first standardized. The input standardization may be implemented based on the raw values of both the markers and survival indicators from a cohort of subjects, such as a relapse event, a death event, and are standardized to ensure consistency and comparability across different scales and units of measurement. This can be achieved through normalization or z-score transformation.

The method for generating a risk indicator can further comprise a Weight Assignment: Each standardized input can be assigned a weight based on its relative importance or contribution to the prognosis, as determined through prior clinical research, expert consensus, or statistical analysis such as regression coefficients. In an embodiment of the invention, cut-off value(s) of a marker raw values may be determined by generating ranges regrouping a subset of marker raw values. For example, a subset can comprise a range of marker raw values representing a percentage of the cohort population. Based on the marker raw values distribution one or more subsets can be generated, thus allowing to determine one or several cut-off value(s), a cut-off value being the maximum value of a first subset of marker raw values and the minimum value of a second subset of marker raw values.

The method further comprises a step of calculating a score by summing the products of each standardized input and its predetermined weight. Mathematically, this can be represented by the following formula:

$$\text{Prognostic Score} = \sum (Wi \times Xi)$$

where Wi is the weigh value and Xi is the Standardized Value of the marker.

Based on the generated subsets and cut-off value(s), a predetermined weight may be associated with a value of the marker based on which subset the value may correspond.

Then, the method may further include a step of associating survival indicators from the cohort of subjects, for example relapse event or a death occurrence over time for each generated subset.

The method thus allows to identify a correlation between the survival indicators and the raw values of the markers to generate groups of subjects with similar survival indicators.

The method also comprises a step of risk stratification wherein a final prognostic score is used to stratify patients into different risk categories (e.g., low, intermediate, high risk) based on predetermined cutoff values. These categories help predict the likelihood of a bad clinical course in DLBCL patients.

Validation and Calibration: The predictive accuracy and reliability of the algorithm are validated against independent patient datasets, and adjustments are made to the weights and cutoff values as necessary to improve prognostic performance.

This computational approach allows for the dynamic integration of multiple factors affecting DLBCL prognosis, providing a more nuanced and individualized risk assessment compared to traditional methods. Additionally, the algorithmic framework facilitates easy updates and refinements as new data become available or as understanding of the disease evolves.

Alternatively, one can use machine learning or other statistical approach like Multivariate Regression Analysis to predict a prognosis characterizing a bad clinical course.

As explained above, the ability to accurately prognose the risk of relapse or a degraded survival expectations allow to implement as early as possible second- or third-line treatments and implement disease management solution.

Accordingly, another object the invention relates to a method of treating a diffuse large B-cell lymphoma in a subject diagnosed as suffering or having a risk of relapse comprising:

- from at least one sample of biological fluid :

    ○ an identification step, in the at least one sample of biological fluid, of at least two markers selected from : 2-aminobutyrate or its acid derivative thereof, 2-hydroxybutyrate or its acid derivative thereof, 3-hydroxybutyrate or its acid derivative thereof, LDL-1 lipoprotein, LDL-2 lipoprotein, LDL-1-CH lipoprotein, LDL-1 lipoprotein phospholipids, the Apo-B subfraction of LDL-1 lipoprotein, LDL-1 lipoprotein triglycerides, LDL-2 lipoprotein triglycerides, LDL-3 lipoprotein triglycerides, formic acid and acetyl acetic acid, identifying said at least two markers being the prognosis of, or a risk of, a bad clinical course of the diffuse large B-cell lymphoma in the subject, and

- a step of adapting the treatment of said subject, including in particular an adaptation of the monitoring rhythm, the administration of an autologous stem cell transplant, the administration of immunomodulator agents (e.g. Golcadomide or Lenalidomide) in combination or not with R-CHOP treatment, administration of an immunotherapy, for example a CAR-T cell therapy (e.g. anti-CD19 or Allo-CART), administration of antibodies (e.g. bispecific anti-CD3 and anti-CD19 antibodies such as blinatumomab), and/or the administration of palliative chemotherapy

An adaptation of the monitoring rhythm encompasses the increase of frequency of the biological analyses or screening tests to detect DLBCL, regarding the usual frequency applied to patients having suffered from DLBCL. In a particular embodiment it can comprises also the monitoring of the at least two markers of the invention as exposed above.

In a particular embodiment, an adaptation of the treatment more particularly submitting the subject identified with a bad clinical course of the diffuse large B-cell lymphoma or a risk thereof to a therapy that is different form the first line therapy or a therapy different from the one he has been treated with.

According to another aspect, described in line with figure 9, the invention is about a system 1 for establishing the prognosis of a subject diagnosed as suffering or having suffered from a diffuse large B-cell lymphoma from at least one sample of biological fluid 10, the system comprising an identification device 20, at least one processor 30 and a referenced DLBCL sample database 40.

According to the system 1 of the invention, the identification device 20 is configured to determine the composition D1 of the biological fluid sample 10, more particularly the identification device 20 is configured to determine markers selected from : 2-aminobutyrate or its acid derivative thereof, 2-hydroxybutyrate or its acid derivative thereof, 3-hydroxybutyrate or its acid derivative thereof, LDL-1 lipoprotein, LDL-2 lipoprotein, LDL-1-CH lipoprotein, LDL-1 lipoprotein phospholipids, the Apo-B subfraction of LDL-1 lipoprotein, LDL-1 lipoprotein triglycerides, LDL-2 lipoprotein triglycerides, LDL-3 lipoprotein triglycerides, formic acid and acetyl acetic acid.

In the invention, the said composition comprises at least two of the previously mentioned markers. As a non-limitative example, the identification device 20 can be a 600 MHz IVDr NMR system (Bruker NEO console) or any kind of device suitable for determining presence and level of the previously mentioned markers.

In the invention, the system 1 comprises a referenced DLBCL sample database 40 comprising:

   ◦ plasma levels, of each sample, of markers : 2-aminobutyrate or its acid derivative thereof, 2-hydroxybutyrate or its acid derivative thereof, 3-hydroxybutyrate or its acid derivative thereof, LDL-1 lipoprotein, LDL-2 lipoprotein, LDL-1-CH lipoprotein, LDL-1 lipoprotein phospholipids, the Apo-B subfraction of LDL-1 lipoprotein, LDL-1 lipoprotein triglycerides, LDL-2 lipoprotein triglycerides, LDL-3 lipoprotein triglycerides, formic acid and acetyl acetic acid,
   ◦ group 41 of referenced DLBCL samples, each group comprising all referenced DLBCL samples for which plasma levels of at least two corresponding markers are associated to a same survival indicator value over time.

For examples, the group 41 can include one or more group such as the one previously mentioned in line with the step of risk stratification wherein a final prognostic score is used to stratify patients into different risk categories (e.g., low, intermediate, high risk) based on predetermined cutoff values.

The system 1 according to the invention further comprises at least one processor 30 configured to calculate a concentration for each marker of the at least two markers identified in the composition.

Indeed, since the identification device 20 allows to determine the plasma levels of the biological sample, the processor 30 may be configured to calculate the concentrations of each marker based on their respective plasma levels.

Moreover, the processor 30 is further configured to compare the calculated concentration of each identified marker to respective predetermined level thresholds to determine an upward increase of each identified marker.

More particularly, the processor 30 may be configured to determine an upward variation of each identified markers from respective predetermined level thresholds.

Preferably, the level thresholds may correspond to the predetermined cutoff values generated from the risk stratification step.

In the invention, the processor 30 is further configured to determine, from the referenced DLBCL sample database 40, at least one sample comprising the same markers, of the at least two markers identified in the composition, for which the upward increase relative to the respective predetermined level thresholds is the closest.

Upon identifying the closest sample from the referenced DLBCL sample database 40, the processor 30 is configured to generate a risk indicator RF1. As previously mentioned, the referenced DLBCL database 40 comprises dataset markers from DLBCL patient samples which are categorized in different groups that may be constructed based on a correlation of an observed survival indicator and at least two markers of the previously mentioned list of markers.

Thus, the risk indicator RF1 is based on the survival indicator associated to the group from which the at least one sample comprising the same markers is determined.

As non-limitative examples, the risk indicator RF1 may describe the survival indicator considered over a period of time, such as the overall survival over 5 years, associated with a probability that the event described by the considered survival indicator occurs in the period of time.

Further aspects and advantages of the invention will be disclosed in the following examples, which should be considered as illustrative.

## EXAMPLES

## MATERIAL AND METHODS

### *In Vitro* Diagnostic by NMR Analysis

#### Sample preparation

A total of 154 biobanked plasmas from the DLBCL REMARC cohort were thawed at room temperature for 30 min. The next steps were performed according to the Bruker IVDr NMR SOP as described elsewhere in detail (2). In brief, 350 $\mu$L of Bruker Plasma Buffer and 350 $\mu$L of plasma were added into a 1.5 mL Eppendorf tube and gently shaken, and then, 600 $\mu$L of the mixture was transferred into a 5 mm NMR tube for measurement.

Spectral Acquisition and Quantification by 1H NMR Spectroscopy

A total of 154 plasma-EDTA samples were analyzed on a 600 MHz IVDr NMR system (Bruker NEO console) operated with double resonance (BBI) room temperature 5 mm probe with z-gradients at 310 K. Standard nuclear Overhauser spectroscopy experiment (1D-NOESY) used in metabolomics was run over 4 min, to quantify 38 polar metabolites and 112 lipoproteins by automated quantification of small molecule metabolites (B.I.QUANT-PSTM) and Bruker IVDr Lipoprotein Subclass Analysis (B.I.LISATM), respectively (2).

#### Markers identification

Over the total of 154 biobanked plasmas from the DLBCL REMARC cohort (1) the presence or absence of the markers and the related ranges (minimum measured value and maximum measured value) of concentrations of each marker have been determined by automated quantification through the Bruker IVDr Platform, each biological sample comprised at least two of the thirteen markers listed in table 1 below.

[Table 1]

| Markers of the invention | CAS number | Value range | Units |
|---|---|---|---|
| 2-aminobutyrate | 2835-81-6 | 0-114 | mmol/L |
| 2-hydroxybutyrate | 565-70-8 | 0-584 | mmol/L |
| 3-hydroxybutyrate | 300-85-6 | 0-591 | mmol/L |
| LDL-1 lipoprotein | n.a | 88,22-1483,27 | nmol/L |
| LDL-2 lipoprotein | n.a | 69,68-597,07 | nmol/L |
| LDL-1-CH lipoprotein | n.a | 5,69-109,48 | mg/dL |
| LDL-1 lipoprotein phospholipids | n.a | 5,01-75,43 | mg/dL |
| Apo-B subfraction of LDL-1 lipoprotein | n.a | 4,85-81,58 | mg/dL |
| LDL-1 lipoprotein triglycerides | n.a | 2,55-82,47 | mg/dL |
| LDL-2 lipoprotein triglycerides | n.a | 1,31-29,68 | mg/dL |
| LDL-3 lipoprotein triglycerides | n.a | 1,03-17,47 | mg/dL |
| formic acid | 64-18-6 | 13-154 | mmol/L |
| acetyl acetic acid | 541-50-4 | 0-576 | mmol/L |

*Statistical Analysis*

X-tile 3.6.1 software (Yale University, New Haven, CT) was used to find optimal dismal outcome-based cut-off values for individual NMR parameter, outcome being either overall survival (OS) or progression free survival (PFS). Kaplan-Meier estimates of OS and PFS were further performed by using the log-rank test (Mantel-Cox) using GraphPad Prism. A value of $p=0.05$ was considered as statistically significant with the following degrees: * $p<0.05$; ** $p<0.01$; *** $p<0.001$.

**RESULTS**

**THE PROGNOSIS OF, OR A RISK OF, A BAD CLINICAL COURSE OF THE DIFFUSE LARGE B-CELL LYMPHOMA (DLBCL) IN SUBJECTS IS DETERMINED BY MARKERS PRESENCE**

**2-aminobutyrate and** LDL-1 lipoprotein **plasma levels are associated with worse overall survival in DLBCL relapse subjects.**

Figure 1 shows the probability of progression free survival based on the 2-aminobutyrate and LDL-1 lipoprotein markers of the cohort subjects over time, each plot (step) representing a progression or a relapse event.
The cohort has been classified based on the 2-aminobutyrate and LDL-1 lipoprotein concentrations which have been measured in every sample. Each plot (step) represents an event of progression or relapse from one of the subjects.
The cohort has been classified under three risk groups generated according to the method for generating a risk indicator of the invention, wherein C1 represents the group with low risk of having a bad clinical course, characterized by biological samples, wherein 2-aminobutyrate concentration is inferior to 52 $\mu$mol/L and LDL-1 lipoprotein concentration is inferior to 227 nmol/L, C2 represents the group with intermediate risk of having a bad clinical course, characterized by biological samples, wherein 2-aminobutyrate concentration is superior to 52 $\mu$mol/L or LDL-1 lipoprotein concentration is superior to 227 nmol/L and C3 represents the group with high risk of having a bad clinical course, characterized by biological samples, wherein 2-aminobutyrate concentration is superior to 52 $\mu$mol/L and LDL-1 lipoprotein concentration is superior to 227 nmol/L.
Figure 2 shows the probability of survival based on the progression free survival (PFS) of the cohort subjects over time based on the International Prognostic Index (IPI).
The cohort has been classified based on the well-known IPI features (age greater than 60 years, elevated serum LDH, performance status, stage of the disease, and number of extranodal sites of disease). Each plot (step) represents an event of progression or relapse from one of the subject.
Three groups have been identified based on the IPI features and classified as C1' represents the group with low risk of having a bad clinical course, C2' represents the group with intermediate risk of having a bad clinical course and C3' represents the group with high risk of having a bad clinical course.
When compared to Figure 1, it is clearly shown that using the above-mentioned markers allows to discriminate at diagnosis much better than IPI, subjects that are at ultra high-risk of having a bad clinical course (C3 curve).
The same observation was made with overall survival as described in Figure 3, which shows the probability of overall survival based on the 2-aminobutyrate and LDL-1 lipoprotein marker of the cohort subjects over time, each plot (step) representing a death event. In comparison, Figure 4 shows the probability of survival based on overall survival (OS) of the cohort subjects over time based on the IPI.
When compared Figure 3, it is clearly shown that using the above-mentioned markers allows to discriminate subjects at high risk of having a bad clinical course (C6 curve) whereas the IPI is much less powerful to identify such subjects with shortened survival, the bad clinical course being represented as the shortened overall survival over time of the subjects.
Figure 5 shows the probability of progression free survival based on the 2-aminobutyrate and 3-hydroxybutyrate markers of the cohort subjects over time, each plot (step) representing a progression or a relapse event.
Figures 6 shows the probability of overall survival based on the 2-aminobutyrate and 3-hydroxybutyrate markers of the cohort subjects over time, each plot (step) representing a death occurrence.
The cohort has been classified based on both the 2-aminobutyrate and 3-hydroxybutyrate markers concentrations which have been measured in every sample.
According to Figures 5 and 6, it is shown that using the above-mentioned markers also allow to discriminate at diagnosis much better from the cohort, those subjects that are at ultra high-risk of having a bad clinical course, showing more frequent relapse event or a shortened death occurrence (as shown by C9 curve and C12 curve) compared to IPI (C3' curve or C6' curve, see Figures 2 and 4).
Figure 7 shows the probability of progression free survival based on the 2-aminobutyrate, LDL-1 lipoprotein and 2-hydroxybutyrate markers of the cohort subjects over time, each plot (step) representing a progression or a relapse event.

Figures 8 shows the probability of overall survival based on the 2-aminobutyrate LDL-1 lipoprotein and 2-hydroxybutyrate markers of the cohort subjects over time, each plot (step) representing a death occurrence.

The cohort has been classified based on the 2-aminobutyrate, LDL-1 lipoprotein and 2-hydroxybutyrate concentrations which have been measured in every sample.

According to Figures 7 and 8, it has been shown that using the above-mentioned markers also allow to discriminate at diagnosis much better from the cohort, those subjects that are at ultra high-risk of having a bad clinical course, more frequent relapse event or a death occurrence (as shown by C15 curve and C18 curve) compared to IPI (C3' curve or C6' curve see Figures 2 and 4).

## References

1. Thieblemont C, Tilly H, Gomes da Silva M, Casasnovas RO, Fruchart C, Morschhauser F, et al. Lenalidomide maintenance compared with placebo in responding elderly patients with diffuse large B-cell lymphoma treated with first-line rituximab plus cyclophosphamide, doxorubicin, vincristine, and prednisone. J Clin Oncol. 2017;35(22):2473-81.

2. Bruker. B.I. Methods. https://www.bruker.com/en/products-and-solutions/mr/nmr-clinical-research-solutions/b-i-methods.html (accessed 27 February 2024).

## Claims

1. An *in vitro* method for establishing the prognosis of a subject diagnosed as suffering or having suffered from a diffuse large B-cell lymphoma from at least one sample of biological fluid, the prognosis method including:

   - an identification step, in the at least one sample of biological fluid, of at least two markers selected from : 2-aminobutyrate or its acid derivative thereof, 2-hydroxybutyrate or its acid derivative thereof, 3-hydroxybutyrate or its acid derivative thereof, LDL-1 lipoprotein, LDL-2 lipoprotein, LDL-1-CH lipoprotein, LDL-1 lipoprotein phospholipids, the Apo-B subfraction of LDL-1 lipoprotein, LDL-1 lipoprotein triglycerides, LDL-2 lipoprotein triglycerides, LDL-3 lipoprotein triglycerides, formic acid and acetyl acetic acid,

   identifying said at least two markers being the prognosis of, or a risk of, a bad clinical course of the diffuse large B-cell lymphoma in the subject.

2. The *in vitro* method for establishing the prognosis according to claim 1, in which the at least two markers comprise 2-aminobutyrate or its acid derivative thereof in combination with at least a marker selected from : 2-hydroxybutyrate or its acid derivative thereof, 3-hydroxybutyrate or its acid derivative thereof, LDL-1 lipoprotein, LDL-2 lipoprotein, LDL-1-CH lipoprotein, LDL-1 lipoprotein phospholipids, the Apo-B subfraction of LDL-1 lipoprotein, LDL-1 lipoprotein triglycerides, LDL-2 lipoprotein triglycerides, LDL-3 lipoprotein triglycerides, formic acid and acetyl acetic acid.

3. The *in vitro* method for establishing the prognosis according to claim 1, in which the at least two markers comprise LDL-1 lipoprotein in combination with at least a marker selected from : 2-aminobutyrate or its acid derivative thereof, 2-hydroxybutyrate or its acid derivative thereof, 3-hydroxybutyrate or its acid derivative thereof, LDL-2 lipoprotein, LDL-1-CH lipoprotein, LDL-1 lipoprotein phospholipids, the Apo-B subfraction of LDL-1 lipoprotein, LDL-1 lipoprotein triglycerides, LDL-2 lipoprotein triglycerides, LDL-3 lipoprotein triglycerides, formic acid and acetyl acetic acid.

4. The *in vitro* method for establishing the prognosis according to claim 1, in which the at least two markers comprise 3-hydroxybutyrate or its acid derivative thereof in combination with at least a marker selected from : 2-aminobutyrate or its acid derivative thereof, 2-hydroxybutyrate or its acid derivative thereof, LDL-1 lipoprotein, LDL-2 lipoprotein, LDL-1-CH lipoprotein, LDL-1 lipoprotein phospholipids, the Apo-B subfraction of LDL-1 lipoprotein, LDL-1 lipoprotein triglycerides, LDL-2 lipoprotein triglycerides, LDL-3 lipoprotein triglycerides, formic acid and acetyl acetic acid.

5. The *in vitro* method for establishing the prognosis according to claim 1, in which the at least two markers comprise 2-aminobutyrate or its acid derivative thereof and LDL-1 lipoprotein optionally in combination with at least a marker selected from : 2-hydroxybutyrate or its acid derivative thereof, 3-hydroxybutyrate or its acid derivative thereof, LDL-2 lipoprotein, LDL-1-CH lipoprotein, LDL-1 lipoprotein phospholipids, the Apo-B subfraction of LDL-1 lipoprotein, LDL-1 lipoprotein triglycerides, LDL-2 lipoprotein triglycerides, LDL-3 lipoprotein triglycerides, formic acid and acetyl acetic acid.

6. The *in vitro* method for establishing the prognosis according to claim 1, in which the at least two markers comprise 2-aminobutyrate or its acid derivative thereof and 3-hydroxybutyrate or its acid derivative thereof optionally in combination with at least a marker selected from : 2-hydroxybutyrate or its acid derivative thereof, LDL-1 lipoprotein, LDL-2 lipoprotein, LDL-1-CH lipoprotein, LDL-1 lipoprotein phospholipids, the Apo-B subfraction of LDL-1 lipoprotein, LDL-1 lipoprotein triglycerides, LDL-2 lipoprotein triglycerides, LDL-3 lipoprotein triglycerides, formic acid and acetyl acetic acid.

7. The *in vitro* method for establishing the prognosis according to claim 1, in which the at least two markers comprise LDL-1 lipoprotein and 3-hydroxybutyrate or its acid derivative thereof optionally in combination with at least a marker selected from : 2-aminobutyrate or its acid derivative thereof, 2-hydroxybutyrate or its acid derivative thereof, LDL-2 lipoprotein, LDL-1-CH lipoprotein, LDL-1 lipoprotein phospholipids, the Apo-B subfraction of LDL-1 lipoprotein, LDL-1 lipoprotein triglycerides, LDL-2 lipoprotein triglycerides, LDL-3 lipoprotein triglycerides, formic acid and acetyl acetic acid.

8. The *in vitro* method for establishing the prognosis according to claim 1, in which the at least two markers comprise 2-aminobutyrate or its acid derivative thereof, LDL-1 lipoprotein and 3-hydroxybutyrate or its acid derivative thereof optionally in combination with at least a marker selected from : 2-hydroxybutyrate or its acid derivative thereof, LDL-2 lipoprotein, LDL-1-CH lipoprotein, LDL-1 lipoprotein phospholipids, the Apo-B subfraction of LDL-1 lipoprotein, LDL-1 lipoprotein triglycerides, LDL-2 lipoprotein triglycerides, LDL-3 lipoprotein triglycerides, formic acid and acetyl acetic acid.

9. The *in vitro* method for establishing the prognosis according to anyone of claims 1 to 8, said method comprising a step of dosing the at least two identified markers and comparing to a predetermined level threshold so as to determine a difference for each of the identified markers, said determination being the prognosis of, or a risk of, a bad clinical course of the diffuse large B-cell lymphoma in the subject.

10. The *in vitro* method for establishing the prognosis according to claim 9 and according to anyone of claims 2, 5, 6 and 8, in which 2-aminobutyrate or its acid derivative thereof is at a concentration superior or equal to 14 $\mu$mol/L.

11. The *in vitro* method for establishing the prognosis according to claim 9 and according to anyone of claims 3, 5, 7 and 8, in which LDL-1 lipoprotein is at a concentration superior or equal to 227 nmol/L

12. The *in vitro* method for establishing the prognosis according to anyone of claims 1 to 11, in which the bad clinical course is selected from : an impaired 5-year survival, an impaired progression-free survival and/or an increased risk of death.

13. The *in vitro* method for establishing the prognosis according to anyone of claims 1 to 12, in which the biological fluid of the at least one sample of biological fluid is selected from : blood, plasma, serum, urine, sweat and/or saliva, more preferably from plasma or urine.

14. The *in vitro* method for establishing the prognosis according to anyone of claims 1 to 13, the subject being a mammal, more preferably a human subject.

15. A method of treating a diffuse large B-cell lymphoma in a subject diagnosed as suffering or having a risk of relapse comprising:

   - from at least one sample of biological fluid :

      ◦ an identification step, in the at least one sample of biological fluid, of at least two markers selected from : 2-aminobutyrate or its acid derivative thereof, 2-hydroxybutyrate or its acid derivative thereof, 3-hydroxybutyrate or its acid derivative thereof, LDL-1 lipoprotein, LDL-2 lipoprotein, LDL-1-CH lipoprotein, LDL-1 lipoprotein phospholipids, the Apo-B subfraction of LDL-1 lipoprotein, LDL-1 lipoprotein triglycerides, LDL-2 lipoprotein triglycerides, LDL-3 lipoprotein triglycerides, formic acid and acetyl acetic acid,

identifying said at least two markers being the prognosis of, or a risk of, a bad clinical course of the diffuse large B-cell lymphoma in the subject, and

- a step of adapting the treatment of said subject, including in particular an adaptation of the monitoring rhythm, the administration of an autologous stem cell transplant, the administration of immunomodulator agents (e.g. Golcadomide or Lenaledomide) in combination or not with R-CHOP treatment, administration of an immunotherapy, for example a CAR-T cell therapy (e.g. anti-CD19 or Allo-CART), administration of antibodies (e.g. bispecific anti-CD3 and anti-CD19 antibodies such as blinatumomab), and/or the administration of palliative chemotherapy.

16. A system (1) for establishing the prognosis of a subject diagnosed as suffering or having suffered from a diffuse large B-cell lymphoma from at least one sample of biological fluid (10), the system (1) comprising an identification device (20), at least one processor (30), a referenced DLBCL sample database (40) comprising :

○ plasma levels, of each sample, of markers : 2-aminobutyrate or its acid derivative thereof, 2-hydroxybutyrate or its acid derivative thereof, 3-hydroxybutyrate or its acid derivative thereof, LDL-1 lipoprotein, LDL-2 lipoprotein, LDL-1-CH lipoprotein, LDL-1 lipoprotein phospholipids, the Apo-B subfraction of LDL-1 lipoprotein, LDL-1 lipoprotein triglycerides, LDL-2 lipoprotein triglycerides, LDL-3 lipoprotein triglycerides, formic acid and acetyl acetic acid,
○ group of referenced DLBCL samples, each group comprising all referenced DLBCL samples for which plasma levels of at least two corresponding markers are associated to a same survival indicator value over time,

in which:

- the identification device (20) is configured to determine the composition (D1) of the biological fluid sample (10), said composition comprising at least two markers selected from : 2-aminobutyrate or its acid derivative thereof, 2-hydroxybutyrate or its acid derivative thereof, 3-hydroxybutyrate or its acid derivative thereof, LDL-1 lipoprotein, LDL-2 lipoprotein, LDL-1-CH lipoprotein, LDL-1 lipoprotein phospholipids, the Apo-B subfraction of LDL-1 lipoprotein, LDL-1 lipoprotein triglycerides, LDL-2 lipoprotein triglycerides, LDL-3 lipoprotein triglycerides, formic acid and acetyl acetic acid,
- the processor (30) is configured to:

○ calculate a concentration for each marker of the at least two markers identified in the composition,
○ compare the calculated concentration of each identified marker to respective predetermined level thresholds to determine an upward increase of each identified marker,
○ determine, from the referenced DLBCL sample database (40), at least one sample comprising the same markers, of the at least two markers identified in the composition, for which the upward increase relative to the respective predetermined level thresholds is the closest,
○ generate a risk indicator (RF1) based on the survival indicator associated to the group from which the at least one sample comprising the same markers is determined.

17. An *in vitro* method for establishing the prognosis of a subject diagnosed as suffering or having suffered from a diffuse large B-cell lymphoma from at least one sample of biological fluid, the prognosis method including:

- an identification step, in the at least one sample of biological fluid, of at least two markers selected from : 2-aminobutyrate or its acid derivative thereof, 2-hydroxybutyrate or its acid derivative thereof, 3-hydroxybutyrate or its acid derivative thereof, LDL-1 lipoprotein, LDL-2 lipoprotein, LDL-1-CH lipoprotein, LDL-1 lipoprotein phospholipids, the Apo-B subfraction of LDL-1 lipoprotein, LDL-1 lipoprotein triglycerides, LDL-2 lipoprotein triglycerides, LDL-3 lipoprotein triglycerides, formic acid and acetyl acetic acid,

identifying said at least two markers being the prognosis of, or a risk of, a bad clinical course of the diffuse large B-cell lymphoma in the subject.

Figure 1

Figure 2

Figure 3

Figure 4

Figure 5

Figure 6

Figure 7

Figure 8

**Figure 9**

Europäisches
Patentamt

European
Patent Office

Office européen
des brevets

## EUROPEAN SEARCH REPORT

**Application Number**

EP 24 18 4216

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | STENSON MARTIN ET AL: "Serum nuclear magnetic resonance-based metabolomics and outcome in diffuse large B-cell lymphoma patients - a pilot study", LEUKEMIA & LYMPHOMA, vol. 57, 17 February 2016 (2016-02-17), pages 1814-1822, XP093225010, Retrieved from the Internet: URL:https://www.tandfonline.com/doi/pdf/10.3109/10428194.2016.1140164> | 1-4,7, 9-17 | INV. G01N33/574 G01N33/92 |
| Y | * abstract * * page 1818, right-hand column; table 2; section "Discussion" * | 1-4,7, 9-17 | |
| Y | YU TIANTIAN ET AL: "Prognostic and therapeutic value of serum lipids and a new IPI score system based on apolipoprotein A-I in diffuse large B-cell lymphoma", AMERICAN JOURNAL OF CANCER RESEARCH, vol. 13, no. 2, 28 February 2023 (2023-02-28), pages 475-484, XP093225006, US ISSN: 2156-6976 Retrieved from the Internet: URL:https://pmc.ncbi.nlm.nih.gov/articles/PMC9989605/pdf/ajcr0013-0475.pdf> * abstract * * page 478; page 482, right-hand column, paragraph 2 * | 1-4,7, 9-17 | |
| A | WO 2022/059785 A1 (ONO PHARMACEUTICAL CO [JP] ET AL.) 24 March 2022 (2022-03-24) * paragraphs [0007], [0008], [0020], [0047], claims 16-18 * | 1-17 | |

**TECHNICAL FIELDS SEARCHED (IPC)**

G01N

-/--

The present search report has been drawn up for all claims

| Place of search | Date of completion of the search | Examiner |
|---|---|---|
| The Hague | 22 November 2024 | Adida, Anne |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 2

Europäisches
Patentamt

European
Patent Office

Office européen
des brevets

**EUROPEAN SEARCH REPORT**

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | CN 117 265 113 A (RUIJIN HOSPITAL SHANGHAI JIAOTONG UNIV SCHOOL MEDICINE) 22 December 2023 (2023-12-22) * the whole document * ----- | 1-17 | |
| A | GAO RUI ET AL: "Low Serum Cholesterol Levels Predict Inferior Prognosis in Diffuse Large B Cell Lymphoma", BLOOD, AMERICAN SOCIETY OF HEMATOLOGY, US, vol. 130, 8 December 2017 (2017-12-08), page 2849, XP086633418, ISSN: 0006-4971, DOI: 10.1182/BLOOD.V130.SUPPL_1.2849.2849 * the whole document * ----- | 1-17 | |

TECHNICAL FIELDS
SEARCHED (IPC)

The present search report has been drawn up for all claims

| Place of search | Date of completion of the search | Examiner |
|---|---|---|
| The Hague | 22 November 2024 | Adida, Anne |

EPO FORM 1503 03.82 (P04C01)

## ANNEX TO THE EUROPEAN SEARCH REPORT
## ON EUROPEAN PATENT APPLICATION NO.

EP 24 18 4216

This annex lists the patent family members relating to the patent documents cited in the above-mentioned European search report.
The members are as contained in the European Patent Office EDP file on
The European Patent Office is in no way liable for these particulars which are merely given for the purpose of information.

22-11-2024

| Patent document cited in search report | Publication date | Patent family member(s) | Publication date |
|---|---|---|---|
| WO 2022059785 A1 | 24-03-2022 | JP WO2022059785 A1<br>TW 202227643 A<br>WO 2022059785 A1 | 24-03-2022<br>16-07-2022<br>24-03-2022 |
| CN 117265113 A | 22-12-2023 | NONE | |

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2017053555 A

**Non-patent literature cited in the description**

- **THIEBLEMONT C** ; **TILLY H** ; **GOMES DA SILVA M** ; **CASASNOVAS RO** ; **FRUCHART C** ; **MORSCH-HAUSER F et al.** Lenalidomide maintenance compared with placebo in responding elderly patients with diffuse large B-cell lymphoma treated with first-line rituximab plus cyclophosphamide, doxorubicin, vincristine, and prednisone. *J Clin Oncol.*, 2017, vol. 35 (22), 2473-81

- **BRUKER. B.I.** *Methods*, 27 February 2024, https://www.bruker.com/en/products-and-solutions/mr/nmr-clinical-research-solutions/b-i-methods.html